# FASCICULE DE BREVET EUROPEEN

(11) **EP 3 175 887 B1**
(45) Date de publication et mention de la délivrance du brevet: **24.06.2020**
(21) Numéro de dépôt: 16197831.7
(22) Date de dépôt: 08.11.2016
(51) Int. Cl.: A61Q 19/06, A61K 8/9789, A61K 8/19, A61K 8/73, A61K 8/9794, A61Q 19/00

(54) **CREME COSMETIQUE DE SUDATION**
KOSMETISCHE ZUBEREITUNG ZUM SCHWITZEN
COSMETIC SWEATING CREAM

(30) Priorité: 01.12.2015 CH 17542015; 03.05.2016 CH 5792016
(43) Date de publication de la demande: 07.06.2017
(73) Titulaire: Bernhard, Denis, 2600 Delemont (CH)
(72) Inventeur: Bernhard, Denis, 2600 Delemont (CH)
(74) Mandataire: P&TS SA (AG, Ltd.)

(56) Documents cités:
- EP-A1- 2 301 558
- DE-U1- 29 616 496
- US-A- 5 139 771
- US-A1- 2003 157 194
- US-A1- 2004 161 435
- US-A1- 2011 034 486
- MARY HELEN LEONARD: "HONEY& CITRUS SALT SCRUB", INTERNET BLOG, 7 mars 2015 (2015-03-07), XP002765065, INTERNET

## Description

### Domaine technique

La présente invention concerne une crème cosmétique de sudation, une méthode d'application de ladite crème et une méthode de fabrication de la dite crème.

### Etat de la technique

Il existe aujourd'hui un certain nombre de crèmes de sudation à appliquer sur la peau d'une zone particulière du corps, typiquement les hanches, le ventre ou encore les bras. Ces crèmes de sudation comprennent fréquemment au moins un composé stimulant comme par exemple la caféine, incorporé dans la crème grâce à des additifs lipophiles comme le glycérol, la vaseline ou encore la paraffine liquide. Les additifs lipophiles confèrent une texture souple à la crème, aident à la solubilisation de la caféine tout en facilitant l'application de la crème sur la peau.

Après application de la crème sur la peau, il est recommandé à l'utilisateur de recouvrir la zone enduite de crème avec un film plastique souple. Après plusieurs heures, l'utilisateur découpe le film et rince la peau pour éliminer les résidus de crème. Il est important de rincer abondamment la peau pour éliminer tous les résidus de crème et laisser ainsi une peau nettoyée et débarrassée d'un maximum d'imperfections. De plus, la présence de restes d'additifs lipophiles issus de la crème pourraient également tacher les vêtements de l'utilisateur.

Alternativement, il existe des crèmes pour une action cosmétique, par exemple anti cellulite, qui comprennent une molécule active. Le document US20110034486 décrit une composition anti cellulite qui comprend notamment un dérivé d'isoflavone qui permet selon les auteurs d'améliorer la lipolyse et ainsi diminuer la cellulite.

Cependant, les crèmes décrites ci-dessus sont souvent très grasses, la présence d'additifs lipophiles compliquant le rinçage puisque ces composés ne sont que très peu solubles dans l'eau. Par exemple, la crème du document DE29616496 est une crème anti cellulite à base de petit lait ou lactosérum et d'émulsifiants. Pour ce type de crème, il est ainsi souvent nécessaire de rincer abondamment la zone qui comprenait la crème, ce qui est fastidieux pour l'utilisateur/utilisatrice. Pour certaines crèmes très grasses, il est même parfois nécessaire d'utiliser un savon pour éliminer entièrement les résidus de crème, ce qui peut aboutir à un dessèchement local, voir à une irritation, à l'opposé de l'effet recherché pour une crème.

Il existe ainsi un besoin d'une crème de sudation non grasse, qui s'étale facilement et se rince aussi facilement.

### Bref résumé de l'invention

Un but de la présente invention est de proposer une crème cosmétique exempt des limitations ou qui minimise les limitations des crèmes cosmétiques connues.

Un but de l'invention est de fournir une crème cosmétique facile à appliquer et facile à rincer après application.

Un autre but de l'invention est de fournir une crème cosmétique qui favorise la sudation de la peau pour lutter contre les imperfections de la peau, qui permette d'adoucir, de rafraichir ou de tonifier la peau.

Un but de l'invention est de fournir une crème cosmétique qui participe à l'amincissement d'un utilisateur qui applique ladite crème sur sa peau.

Selon l'invention, ces buts sont atteints notamment au moyen d'une crème cosmétique de sudation selon la revendication 1.

La crème cosmétique selon la présente invention a un effet uniquement cosmétique en ce qu'elle a pour but d'embellir la peau de l'utilisateur/utilisatrice.

Les inventeurs ont découverts que la crème cosmétique selon l'invention favorise la sudation de la zone corporelle sur laquelle elle est appliquée. Avantageusement, l'augmentation de la sudation peut s'accompagner d'un effet tonifiant et rafraichissant. La crème cosmétique selon l'invention permet aussi un raffermissement et un amincissement de la zone où la crème cosmétique est appliquée.

La crème cosmétique selon l'invention comporte essentiellement des composés hydrophiles ce qui facilite le rinçage avec une solution aqueuse, par exemple de l'eau, après utilisation. La présence d'un ou plusieurs sels combinés aux agrumes assure un nettoyage efficace de la peau, en facilitant la sudation. En effet, les agrumes ont des propriétés rafraichissantes et tonifiantes et sont également efficaces contre les imperfections de la peau comme le point noir ou autre comédons. Les agrumes permettent aussi d'adoucir la peau en particulier les zones rugueuses (genoux, talons, coudes par exemple). Les glucides permettent d'absorber les fluides sécrétés, comme la sueur, de façon à ce qu'ils ne pénètrent pas à nouveau dans la peau.

La crème cosmétique selon l'invention contient essentiellement des produits naturels contrairement aux crèmes existantes ce qui permet de minimiser les effets indésirables associés à certains produits de synthèses.

La crème cosmétique de sudation comprend de l'amidon de maïs. L'amidon de maïs est un glucide naturel qui offre de bonnes capacités d'absorption des liquides.

Dans un mode de réalisation, la crème cosmétique selon l'invention peut comprendre des graisses, fibres ou protéines, mais avec des proportions faibles de sorte que la crème cosmétique n'est pas grasse. Par exemple, les glucides utilisés peuvent comprendre des graisses, protéines ou fibres alimentaires, par exemple à hauteur d'environ 1 % en poids.

L'extrait d'agrumes comprend un jus de citron. Classiquement, les crèmes à base d'agrumes utilisent de la pulpe de fruits ou des fibres ce qui ne facilite pas l'étalement de la crème sur la peau. Il est intéressant de partir de jus d'agrumes car cela permet d'obtenir une crème cosmétique homogène qui ne contient pas de particules solides. En particulier, le jus d'agrumes peut être pasteurisé avant d'être incorporé dans la crème cosmétique. Cela permet de minimiser la présence de pathogènes dans la crème.

La crème cosmétique comprend du chlorure de sodium comme constituant majoritaire ou unique du ou des sels. Le sel utilisé peut comprendre de l'iode ou du fluor, par exemple à l'état de traces inférieur à 0.1 % en poids.

La solution aqueuse est constituée essentiellement d'eau.

Selon un mode de réalisation, la crème cosmétique comprend de l'essence de produit naturel, de préférence de l'essence de plante. Les essences de produits naturels, par exemple les huiles essentielles, parfument la crème et ont aussi des propriétés apaisantes, nourrissantes et réparatrices pour des peaux agressées et irritées.

Dans un mode de réalisation, la crème cosmétique de sudation comprend:
- Entre 12.0 % en poids et 17.0 % en poids d'amidon de maïs;
- Entre 40.0 % en poids et 55.0 % en poids de jus de citrons;
- Entre 30.0 % en poids et 45.0 % en poids de NaCl;
- Entre 0 % en poids et 10.0 % en poids d'eau.

Dans un mode de réalisation, la crème cosmétique de sudation comprend:
- Entre 12.0 % en poids et 16.0 % en poids d'amidon de maïs;
- Entre 43.0 % en poids et 47.0 % en poids de jus de citrons;
- Entre 37.0 % en poids et 41.0 % en poids de NaCl;
- Entre 0.1 % en poids et 3.0 % en poids d'eau.

Dans un mode de réalisation, la crème cosmétique contient une quantité très faible de solution aqueuse. Par exemple, la crème contient entre 0.1 et 1.6 % de solution aqueuse. Avantageusement, la crème cosmétique ne contient pas de solution aqueuse. Les inventeurs ont découvert que lorsque la crème contient une quantité très faible de solution aqueuse, la crème adhère mieux à la peau lors de l'application. Cette observation se vérifie aussi lorsque la crème ne contient pas de solution aqueuse.

Les inventeurs ont aussi découvert qu'une quantité très faible de solution aqueuse permettait d'améliorer la durée de conservation de la crème. Cette observation se vérifie aussi lorsque la crème ne contient pas de solution aqueuse.

L'invention concerne également une méthode de fabrication d'une crème cosmétique de sudation, la méthode comprenant :
i) Préparer une solution initiale en dissolvant au moins 80% en poids de sel dans au moins 30% en poids de la solution aqueuse ou dans au moins 30 % de l'extraits d'agrumes lorsque la crème ne contient pas de solution aqueuse, en maintenant ladite solution initiale sous agitation entre 90°C et 100°C jusqu'à que la solution initiale soit homogène;
ii) Préparer une solution de liaison comprenant :
   ii)1) au moins 90 % en poids de glucides, les extraits d'agrumes et le reste de solution aqueuse, si la crème comprend une solution aqueuse ; ou
   ii)2) au moins 90 % en poids de glucides, et le reste des extraits d'agrumes si la crème ne comprend pas de solution aqueuse ;
   et maintenir ladite solution de liaison ii)1) ou ii)2) sous agitation jusqu'à ce que la solution de liaison soit homogène;
iii) Incorporer progressivement la solution de liaison dans la solution initiale pour préparer une composition de base, la dite solution initiale étant maintenue à une température comprise entre environs 55°C et environs 65°C ;
iv) A l'issu de l'incorporation, chauffer la composition de base sous agitation à une température comprise entre 65°C et 75°C jusqu'à obtenir une crème homogène.

Dans un mode de réalisation, lorsque la crème contient une faible quantité de solution aqueuse, par exemple moins de 15 % en poids, de préférence moins de 10 % en poids, cette faible quantité de solution aqueuse est introduite dans la crème à l'issu de l'incorporation de la solution de liaison dans la solution initiale, c'est-à-dire après l'étape iii). En particulier, cette faible quantité de solution aqueuse est introduite dans l'étape iv).

Dans un mode de réalisation, la crème de sudation comprend une solution aqueuse. Dans ce cas, l'étape i) du procédé selon l'invention comprend la préparation d'une solution initiale en dissolvant au moins 80% en poids de sel dans au moins 30% en poids de la solution aqueuse. La solution initiale est alors appelée saumure. Une méthode de fabrication d'une crème cosmétique de sudation selon l'invention, la crème comprenant une solution aqueuse, peut comprendre les étapes suivantes:
i) Préparer une saumure en dissolvant au moins 80% en poids de sel dans au moins 30% en poids de la solution aqueuse en maintenant ladite saumure sous agitation entre 90°C et 100°C jusqu'à que la saumure soit homogène;
ii) Préparer une solution de liaison comprenant :
   - au moins 90 % en poids de glucides,
   - les extraits d'agrumes et
   - le reste de solution aqueuse
   et maintenir ladite solution de liaison sous agitation jusqu'à ce que la solution de liaison soit homogène ;
iii) Incorporer progressivement la solution de liaison dans la saumure pour préparer une composition de base, la dite saumure étant maintenue à une température comprise entre environs 55°C et environs 65°C ;
iv) A l'issu de l'incorporation, chauffer la composition de base sous agitation à une température comprise entre 65°C et 75°C jusqu'à obtenir une crème homogène.

Dans un mode de réalisation, l'étape i) comprend
i) Préparer une solution initiale en dissolvant au moins 80% en poids de sel dans au moins 50% en poids de la solution aqueuse ou dans au moins 50 % de l'extraits d'agrumes lorsque la crème ne contient pas de solution aqueuse, en maintenant ladite solution initiale sous agitation entre 90°C et 100°C jusqu'à que la solution initiale soit homogène.

Dans la méthode de fabrication selon l'invention, les inventeurs ont découvert que lorsque la solution de liaison est incorporée dans la solution initiale maintenue entre environs 55°C et environs 65°C, cela facilite l'incorporation de la solution de liaison dans la solution initiale sans détériorer les composants de la solution de liaison. A l'issue de l'incorporation, la composition de base chauffée et agitée entre 65°C et 70°C pour homogénéiser et stabiliser la composition de base.

Selon un mode de réalisation, les sels et glucides sont introduits dans la crème cosmétique chacun en deux portions à des étapes différentes de la fabrication de la crème. La première portion en sel comprend par exemple 80% en poids de sel, le reste étant dans la deuxième portion. La première portion en glucides comprend 90 % en poids des glucides, le reste étant dans la deuxième portion. Dans un mode de réalisation, la deuxième portion de sel et la deuxième portion de glucides sont incorporés dans la crème après l'étape iv), dans une crème à température comprise entre 25°C et 50°C. Les inventeurs ont découverts que lorsqu'au moins une partie des glucides n'est pas chauffé à une température supérieure à 50°C, cela améliore sensiblement les capacités d'absorption des fluides de la crème cosmétique, par exemple l'absorption de la sueur. Avantageusement, les inventeurs ont aussi découverts que l'incorporation de la deuxième portion de sel permettait d'obtenir une crème plus concentrée en sel, voir saturée en sel ce qui améliorait son effet de sudation. Cela permet aussi d'obtenir une crème cosmétique plus lisse et onctueuse qui s'étale plus facilement.

Dans un mode de réalisation, la méthode comprend :
- v) Incorporer le reste de sel et le reste de glucide dans la crème.

L'invention concerne également une méthode d'application d'une crème cosmétique comprenant:
i) Appliquer la crème cosmétique de sudation selon l'invention sur une zone d'application de la peau ;
ii) Recouvrir la zone d'application avec un papier film ;
iii) Après au minimum 2 heures d'application, ôter le film et rincer la zone d'application avec une solution aqueuse.

Par exemple, environs 30 à 40 g de crème est appliqué sur une zone telle que les hanches ou le ventre.

Le papier film est par exemple un papier en matière plastique étirable qui s'adapte à la forme qu'il recouvre.

Après au moins 2 heures d'application, la crème cosmétique permet d'obtenir un effet tonifiant, rafraichissant mais aussi un raffermissement et un amincissement de la zone où la crème cosmétique est appliquée. L'utilisateur peut choisir de garder la crème cosmétique plus longtemps, par exemple toute la nuit, pour renforcer l'effet de la crème sur la zone où la crème cosmétique est appliquée.

De manière surprenante, les inventeurs ont découvert que la crème cosmétique selon l'invention a des propriétés mécaniques propre, en particulier lorsque les glucides comprennent de l'amidon de maïs. Il est connu que l'amidon de maïs possède des propriétés rhéoépaississante. Lorsque la crème cosmétique est comprimée par le film sur une partie du corps qui est en mouvement, la crème cosmétique se comporte en partie comme un fluide rhéoépaississant grâce à la présence d'amidon. La viscosité de la crème cosmétique diminue plus la contrainte sur la crème cosmétique est forte. Ainsi, la crème cosmétique absorbe mieux les fluides corporels comme par exemple la sueur.

### Exemple de mode de réalisation de l'invention

Dans un premier exemple, la crème cosmétique sans solution aqueuse selon la présente invention peut être fabriquée selon le protocole suivant :
1) Préparer une solution initiale en dissolvant 30 g. de NaCl dans 30 cl jus de citron, en agitant et en chauffant à 95°C la dite solution initiale jusqu'à ce que la solution initiale soit homogène;
2) Préparer une solution de liaison en mélangeant 13.5 g de fécule de maïs, 20 cl de jus de citron en agitant et en chauffant à 95°C la dite solution de liaison jusqu'à ce que la solution de liaison soit homogène ;
3) Incorporer progressivement la solution de liaison dans la solution initiale pour préparer une composition de base, la dite solution initiale étant à une température de 65°C ;
4) A l'issu de l'incorporation, chauffer la composition de base sous agitation à une température de 75°C pendant 1 à 2 minutes jusqu'à obtenir une crème homogène.
5) Laisser la crème revenir à température inférieure à 50°C et incorporer 1.5 g. de fécule de maïs et 5 g. de NaCl.
La crème fabriquée selon cet exemple est prête à être appliquée sur la peau d'un utilisateur.

Dans un deuxième exemple, la crème cosmétique comprenant 34 % en poids de solution aqueuse selon la présente invention peut être fabriquée selon le protocole suivant :
1) Préparer une saumure en dissolvant 24 g. de NaCl dans 23 cl d'eau en agitant et en chauffant à 95°C la dite saumure jusqu'à ce que la saumure soit homogène;
2) Préparer une solution de liaison en mélangeant 8.5 g de fécule de maïs, 28.5 cl de jus de citrons pasteurisé, 11 cl d'eau, en agitant et en chauffant à 95°C la dite solution de liaison jusqu'à ce que la solution de liaison soit homogène;
3) Incorporer progressivement la solution de liaison dans la saumure pour préparer une composition de base, la dite saumure étant à une température de 65°C;
4) A l'issu de l'incorporation, chauffer la composition de base sous agitation à une température de 75°C pendant 1 à 2 minutes jusqu'à obtenir une crème homogène.
5) Laisser la crème revenir à température inférieure à 50°C et incorporer 1 g. de fécule de maïs et 4 g. de NaCl.
La crème fabriquée selon cet exemple est prête à être appliquée sur la peau d'un utilisateur.

## Revendications

1. Crème cosmétique de sudation comprenant :
- Entre 12.0 % en poids et 17.0 % en poids d'amidon de maïs;
- Entre 40.0 % en poids et 55.0 % en poids de jus de citrons;
- Entre 30.0 % en poids et 45.0 % en poids de NaCl;
- Entre 0 % en poids et 10.0 % en poids d'eau.

2. Crème cosmétique de sudation selon la revendication 1 dans laquelle le jus de citrons est pasteurisé.

3. Crème cosmétique de sudation selon l'une des revendications précédentes comprenant de l'essence de produit naturel, de préférence de l'essence de plante.

4. Méthode de fabrication d'une crème cosmétique de sudation selon l'une des revendications 1 à 3, la méthode comprenant:
i) Préparer une solution initiale en dissolvant au moins 80% en poids de NaCl dans au moins 30% en poids de l'eau ou dans au moins 30 % de jus de citrons lorsque la crème ne contient pas d'eau, en maintenant ladite solution initiale sous agitation entre 90°C et 100°C jusqu'à que la solution initiale soit homogène;
ii) Préparer une solution de liaison comprenant :
ii)1) au moins 90 % en poids de l'amidon de maïs, le jus de citrons et le reste d'eau, si la crème comprend de l'eau ; ou
ii)2) au moins 90 % en poids d'amidon de maïs, et le reste de jus de citrons si la crème ne comprend pas d'eau;
et maintenir ladite solution de liaison ii)1) ou ii)2) sous agitation jusqu'à ce que la solution de liaison soit homogène ;
iii) Incorporer progressivement la solution de liaison dans la solution initiale pour préparer une composition de base, la dite solution initiale étant maintenue à une température comprise entre environs 55°C et environs 65°C ;
iv) A l'issu de l'incorporation, chauffer la composition de base sous agitation à une température comprise entre 65°C et 75°C jusqu'à obtenir une crème homogène.

5. Méthode selon la revendication précédente comprenant l'étape suivante, après l'étape iv) :
- v) Incorporer le reste de NaCl et le reste d'amidon de maïs dans la crème.

6. Méthode d'application d'une crème de sudation selon l'une des revendications 1 à 3 comprenant :
i) Appliquer la crème cosmétique de sudation selon l'une des revendications 1 à 3 sur une zone d'application de la peau ;
ii) Recouvrir la zone d'application avec un papier film ;
iii) Après au minimum 2 heures d'application, ôter le film et rincer la zone d'application avec une solution aqueuse.

## Patentansprüche

1. Kosmetische Schwitzcreme, bestehend aus :
- Zwischen 12,0 Gewichtsprozent und 17,0 Gewichtsprozent Maisstärke
- Zwischen 40,0 Gewichtsprozent und 55,0 Gewichtsprozent Zitronensaft;
- Zwischen 30,0 Gewichtsprozent und 45,0 Gewichtsprozent NaCl;
- Zwischen 0 Gewichtsprozent und 10,0 Gewichtsprozent Wasser.

2. Kosmetische Schwitzcreme nach Anspruch 1, deren Zitronensaft pasteurisiert ist.

3. Eine kosmetische Schwitzcreme gemäß einem der vorstehenden Ansprüche, die Naturproduktessenz, vorzugsweise Pflanzenessenz, enthält.

4. Verfahren zur Herstellung einer kosmetischen Schwitzcreme nach einem der Ansprüche 1 bis 3, umfassend:
i) Zubereitung einer Ausgangslösung durch Auflösen von mindestens 80 Gewichtsprozent Salz in mindestens 30 Gewichtsprozent Wasser oder in mindestens 30 Gewichtsprozent Zitronensaft, sofern die Creme kein Wasser enthält, wobei die Ausgangslösung unter Rühren zwischen 90°C und 100°C gehalten wird, bis die Ausgangslösung homogen ist;
ii) Zubereitung einer Bindungslösung, bestehend aus:
ii)1) mindestens 90 Gewichtsprozent Maisstärke, Zitronensaft und der Rest Wasser, wenn die Creme Wasser enthält; oder
ii)2) mindestens 90 Gewichtsprozent Maisstärke, und der Rest Zitronensaft, wenn die Creme kein Wasser enthält; und beständiges Rühren der Bindungslösung ii)1) oder ii)2) bis die Lösung homogen ist;
iii) schrittweises Einarbeiten der Bindungslösung in die Ausgangslösung zur Zubereitung einer Grundzusammensetzung, wobei besagte Ausgangslösung auf einer Temperatur zwischen etwa 55 °C und ungefähr 65 °C gehalten wird;
(iv) Nach dem Einarbeiten, Erhitzen der Grundzusammensetzung auf eine Temperatur zwischen 65 °C und 75 °C unter Rühren bis eine homogene Creme zu erhalten ist.

5. Verfahren nach dem vorhergehenden Anspruch, umfassend den folgenden Schritt nach Schritt iv) :
v) Einarbeiten des restlichen NaCl und der restlichen Maisstärke in die Creme.

6. Verfahren zum Auftragen einer Schwitzcreme nach einem der Ansprüche 1 bis 3, umfassend:
i) Auftragen der kosmetischen Schwitzcreme nach einem der Ansprüche 1 bis 3 auf ein Anwendungsbereich der Haut;
ii) Abdecken des Anwendungsbereichs mit einem Filmpapier;
iii) Nach mindestens 2-stündigem Auftragen wird der Film entfernt und die Applikationsfläche mit einer wässrigen Lösung gespült.

## Claims

1. Cosmetic sudation cream comprising:
- Between 12.0 % in weight and 17.0 % in weight of corn starch;
- Between 40.0 % in weight and 55.0 % in weight of lemon juice;
- Between 30.0 % in weight and 45.0 % in weight of NaCl;
- Between 0 % in weight and 10.0 % in weight of water.

2. Cosmetic sudation cream according to claim 1, wherein the lemon juice is pasteurised.

3. Cosmetic sudation cream according to one of the previous claims, comprising the essence of a natural product, preferably a plant essence.

4. Manufacturing method of a cosmetic sudation cream according to one of the claims 1 to 3, the method comprising:
i) Prepare an initial solution by dissolving at least 80% in weight of NaCl in at least 30% in weight of water or at least 30 % of lemon juice when the cream does not contain any water, while maintaining said initial solution in agitation between 90°C and 100°C until the initial solution becomes homogeneous;
ii) Prepare a binding solution comprising:
ii)1) at least 90 % in weight of corn starch, the lemon juice and the rest of the water, if the cream comprises water; or
ii)2) at least 90 % in weight of corn starch, and the rest of the lemon juice if the cream does not comprise water;
and maintain said binding solution ii)1) or ii)2) in agitation until the binding solution is homogeneous;
iii) Progressively incorporate the binding solution into the initial solution to prepare a basic composition, said initial solution being kept at a temperature of between approximately 55°C and approximately 65°C ;
iv) At the end of the incorporation, heat the basic composition in agitation to a temperature of between 65°C and 75°C until a homogeneous cream is obtained.

5. Method according to the previous claim comprising the following step, after step iv):
- v) Incorporate the rest of the NaCl and the rest of the corn starch into the cream.

6. Application method for a sudation cream according to one of the claims 1 to 3 comprising:
i) Apply the cosmetic sudation cream according to one of the claims 1 to 3 on an application area of the skin;
ii) Cover the application area with cling film;
iii) After at least 2 hours of application, remove the film and rinse the application area with an aqueous solution.
